# EUROPEAN PATENT APPLICATION

(11) **EP 2 213 281 A1**
(43) Date of publication of application: **04.08.2010**
(21) Application number: 09001406.9
(22) Date of filing: 02.02.2009
(51) Int. Cl.: A61K 9/20, A61K 47/38, C07D 239/94, A61K 31/517

(54) **Pharmaceutical composition comprising N-(3-ethynylphenyl)-6,7-bis(2-methoxyethoxy)-4-quinazolinamine hydrochloride**

(71) Applicant: Ratiopharm GmbH, 89079 Ulm (DE)
(72) Inventor: Brück, Sandra, 85570 Ottenhofen (DE); Holfinger, Konstantin, 88326 Aulendorf (DE)
(74) Representative: Teipel, Stephan

(57) **Abstract**

The present invention relates to a pharmaceutical composition comprising N-(3-ethynylphenyl)-6,7-bis(2-methoxyethoxy)-4-quinazolinamine hydrochloride as active pharmaceutical ingredient and a process for the preparation of such compositon.

## Description

### Field of the Invention

The present invention relates to a pharmaceutical composition comprising N-(3-ethynylphenyl)-6,7-bis(2-methoxyethoxy)-4-quinazolinamine hydrochloride as active pharmaceutical ingredient and a process for the preparation of such compositon.

### Background of the invention

The compound N-(3-ethynylphenyl)-6,7-bis(2-methoxyethoxy)-4-quinazolinamine (INN: Erlotinib) is useful in the treatment of hyperproliferative disorders, such as cancers, in mammals. Erlotinib HCl has the following formula (I):

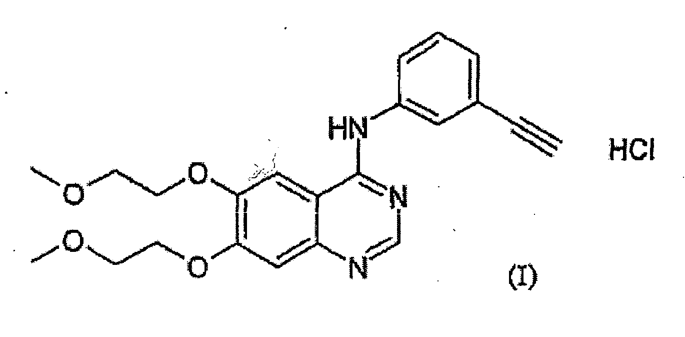

In WO 96/30347, which is incorporated herein by reference in its entirety, the preparation of N-(3-ethynylphenyl)-6,7-bis(2-methoxyethoxy)-4-quinazolinamine hydrochloride as well as the uses of this compound have been disclosed. In particular, this compound is an inhibitor of tyrosine kinase enzymes, namely as epidermal growth factor receptors and can be used for the treatment and/or prevention of diseases which are associated with tyrosine kinase enzymes such as epidermal growth factor receptors, such as cancer, particularly non small cell lung cancer, colorectal cancer, refractory non small cell lung cancer, pancreatic cancer, ovarian cancer, breast cancer, glioma, head cancer or neck cancer.

Recently, two different polymorphs of N-(3-ethynylphenyl)-6,7-bis(2-methoxyethoxy)-4-quinazolinamine hydrochloride, designated as polymorph A and polymorph B, have been disclosed in WO 01/34574, which is incorporated herein by reference. Polymorph B was found to be thermodynamically more stable than polymorph A. This document therefore also discloses a method comprising the step of recrystallisation of a compound comprising both polymorph A and polymorph B into the more stable polymorph B. The more stable polymorph B is further used in the preparation of pharmaceutical compositions.

A further polymorph of the compound N-(3-ethynylphenyl)-6,7-bis(2-methoxyethoxy)-4-quinazolinamine hydrochloride, namely polymorph E is disclosed in WO 2004/072049. This document further teaches that while polymorph B is thermodynamically more stable than polymorph A, polymorph A exhibits a better solubility and dissolution rate than polymorph B. Polymorph E is said to unexpectedly exhibit the desired increased solubility and dissolution rate with respect to polymorph B and an increased thermodynamic stability compared to polymorph A.

Without disclosing any specific polymorph of N-(3-ethynylphenyl)-6,7-bis(2-methoxyethoxy)-4-quinazolinamine hydrochloride WO 2006/110811 relates to the problem that conventional Erlotinib HCl tablets are only very slightly soluble in water at 37°C. Therefore, the dissolution of conventional Erlotinib HCl tablets is said to be reduced in the fasted state as compared to the fed state. Thus, Erlotinib HCl has limited bioavailability in the fasted state as compared to the fed state, which limits the therapeutic outcome for all treatments requiring Erlotinib HCl. To overcome this problem a nanoparticulate Erlotinib HCl composition comprising particles of the active pharmaceutical ingredient having an effective average particle size of less than 2000 nm and at least one surface stabilizer is suggested. The surface stabilizer is present to stabilize the nanoparticulate form of the composition. A wide range of surface stabilizers is disclosed, including various polymers, low molecular weight oligomers, natural products, and surfactants. The surface stabilizers include non-ionic, ionic anionic, cationic, and zwitterionic compounds or surfactants. Sodium lauryl sulfate is exemplified among a large list of further compounds.

Nanoparticles are difficult to prepare, costly, and difficult to handle when preparing pharmaceutical dosage forms. Therefore, there is a need for further pharmaceutical compositions comprising Erlotinib HCl which overcome the above problems of the prior art, in particular with respect to preparation and handling characteristics, solubility, bioavailability and stability.

It has now surprisingly been found that the above problems can be overcome by providing a pharmaceutical composition comprising polymorph A of N-(3-ethynylphenyl)-6,7-bis(2-methoxyethoxy)-4-quinazolinamine hydrochloride. Thus, the present invention relates to a pharmaceutical composition comprising N-(3-ethynylphenyl)-6,7-bis(2-methoxyethoxy)-4-quinazolinamine hydrochloride in a thermodynamically stable form.

### Description of the invention

Erlotinib HCl can be synthesized using techniques well-known in the art. A synthesis of Erlotinib is disclosed, for example, in WO 96/30347.

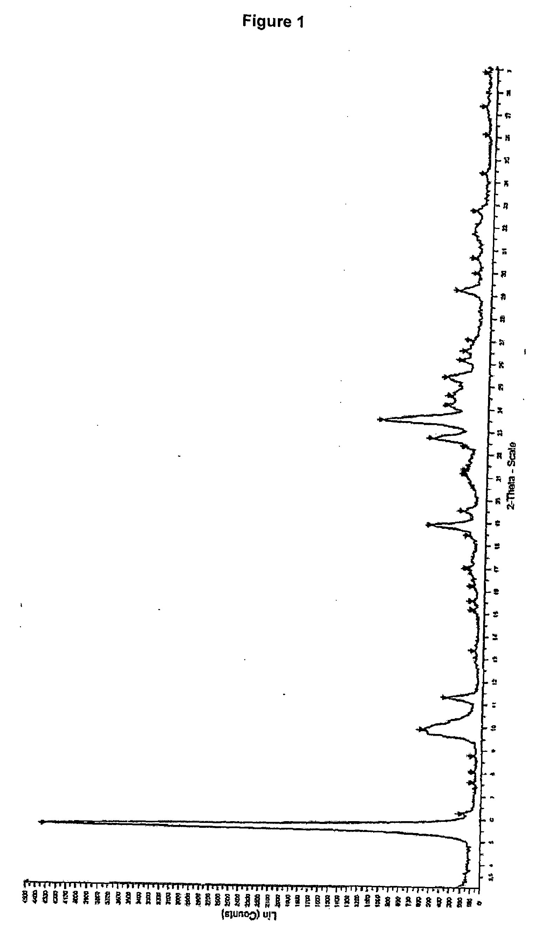

Polymorph A of Erlotinib HCl and a method of its preparation is disclosed in WO 01/34574. The X-ray powder diffraction pattern of polymorph A of Erlotinib HCl is shown in attached figure 1. 2-Theta values of characteristic peaks taken from the X-ray powder diffraction pattern of polymorph A of Erlotinib HCl and their relative intensities are summarized in the following table 1.

**Table 1:**

| Anode: Cu - Wavelength 1: 1.54056 Wavelength 2: 1.54439 (Rel Intensity: 0.500) Range#1 - Coupled: 3.000 to 40.000 StepSize: 0.040 Step Time: 1.00 Smoothing Width: 0.300 Threshold: 1.0 | | | | | | | | | |
|---|---|---|---|---|---|---|---|---|---|
| **2-Theta** | **I(rel)** | **2-Theta** | **I(rel)** | **2-Theta** | **I(rel)** | **2-Theta** | **I(rel)** | **2-Theta** | **I(rel)** |
| 5.579 | 100.0 | 13.340 | 1.7 | 19.517 | 4.8 | 24.594 | 8.2 | 30.673 | 3.5 |
| 6.165 | 3.9 | 15.135 | 2.1 | 21.152 | 4.7 | 25.398 | 9.3 | 32.759 | 3.7 |
| 7.522 | 1.5 | 15.534 | 2.3 | 21.320 | 4.4 | 26.173 | 6.0 | 34.440 | 1.8 |
| 8.006 | 1.2 | 16.193 | 2.4 | 22.360 | 4.7 | 26.572 | 5.3 | 36.154 | 1.3 |
| 8.696 | 1.4 | 16.991 | 3.6 | 22.703 | 12.4 | 27.080 | 4.2 | 37.404 | 2.2 |
| 9.841 | 13.1 | 18.447 | 3.5 | 23.502 | 24.2 | 29.240 | 7.1 | 38.905 | 1.7 |
| 11.251 | 7.8 | 18.862 | 12.2 | 24.175 | 8.8 | 30.007 | 3.0 | | |

Thus, polymorph A of Erlotinib HCl is characterized by having characteristic peaks in an X-ray powder diffraction pattern when measured with Cuk_{α} radiation expressed in degrees 2-theta at approximately 5.6, 9.8, 18.9 and 23.5.

The term "pharmaceutical composition" refers to single dosage forms, such as tablets, capsules, pellets, etc., as well as powders or granules which are used in the preparation of single dosage forms. Where it is referred to the total weight of the pharmaceutical composition and the pharmaceutical composition is in a single dosage form, the total weight is the weight of the single dosage form excluding, if applicable, the weight of any coating or capsule shell.

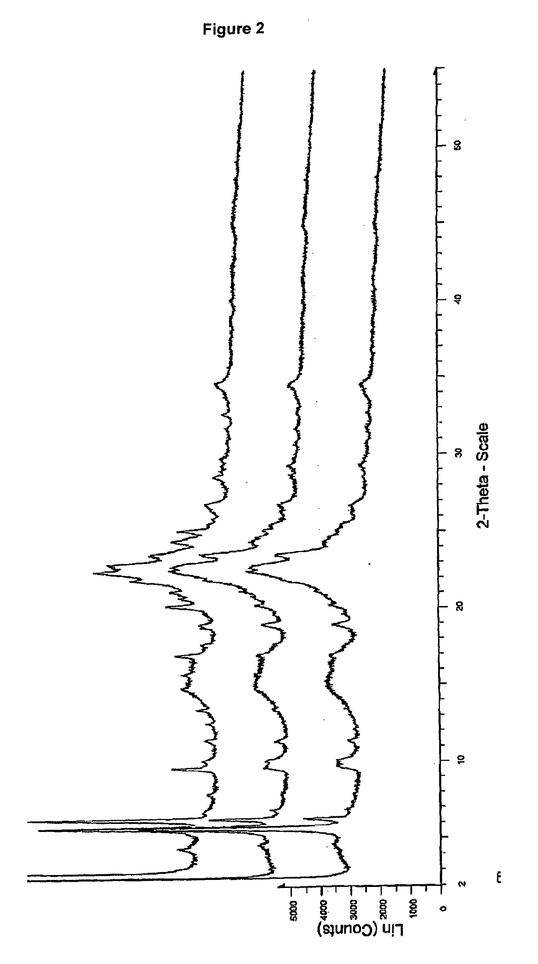

It is known form WO 01/34574 and WO 2004/072049 that polymorph A of Erlotinib HCl is thermodynamically unstable. This is confirmed by attached figure 2 which shows the X-ray powder diffraction (XRPD) pattern of a mixture of 90 parts by weight polymorph A and 10 parts by weights polymorph B during storage for 0, 4 and 12 weeks respectively (from bottom to top). From the diffraction patterns it can be seen that the characteristic peaks of polymorph A are decreasing while the characteristic peaks of polymorph B are increasing over time. For example, the relative intensities of the polymorph A peak at a 2-theta angle of 5.6 are decreasing from the bottom to the top while the relative intensities of the polymorph B peak at a 2-theta angle of 6.3 are significantly increasing from the bottom to the top, i.e. during storing for 0, 4 and 12 weeks, respectively. This confirms the prior art knowledge that under conventional conditions polymorph A is thermodynamically unstable and converts into polymorph B.

Contrary to this prior art knowledge, the present invention is based on the surprising finding that polymorph A of Erlotinib HCl can be incorporated into a pharmaceutical composition in a thermodynamically stable form. "Thermodynamically stable" in the sense of the present invention means that in the pharmaceutical composition Erlotinib HCl maintains its polymorphic form A even under harsh storage conditions of 75 % relative humidity and 40°C for at least 12 weeks, preferably for at least 12 months. Maintaining its polymorphic form A therefore means that polymorph A of Erlotinib HCl during storage is not converted into any other polymorph of Erlotinib HCl, in particular not into polymorph B.

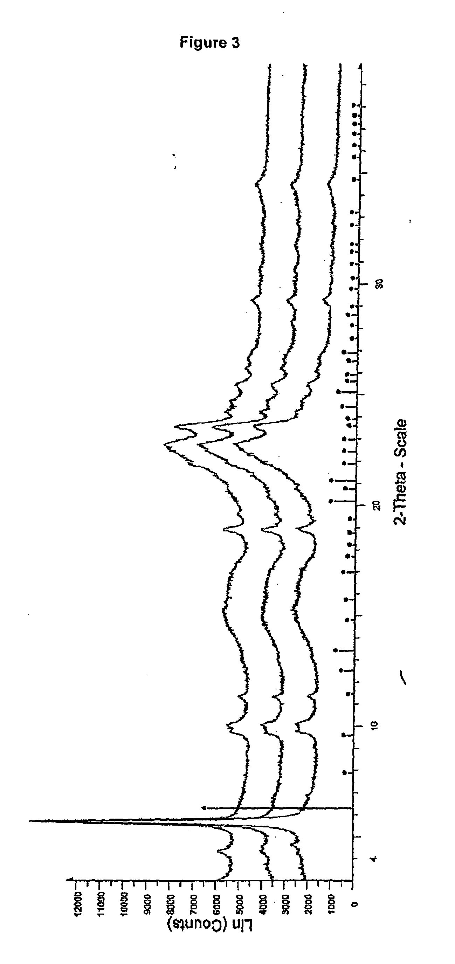

The thermodynamic stability of polymorph A of Erlotinib HCl in the pharmaceutical compositon of the present invention is confirmed by attached figure 3 which shows the XRPD patterns of grounded tablets of the present invention at time 0, after 4 weeks and after 12 weeks storage at 75 % relative humidity and 40°C (from bottom to top). It is evident that the characteristic peaks of polymorph A of Erlotinib HCl do not change in their intensities. Moreover, none of the characteristic peaks of polymorph B of Erlotinib HCl (shown as lines and dots at the bottom of the diagram) occurs in the XRPD patterns of the tablet. This confirms that polymorph A of Erlotinib HCl is thermodynamically stable in the pharmaceutical composition of the present invention and in particular that polymorph A is not converted into polymorph B during storage over at least 12 weeks.

Furthermore, it has surprisingly been found that in one embodiment of the present invention, polymorph A of Erlotinib HCl can be thermodynamically stabilized by the addition of a hydrophilic excipient to the pharmaceutical composition. Further surprising it was found that the addition of a hydrophilic excipient does not only stabilize the polymorphic form of the active pharmaceutical ingredient, but the hydrophilic excipient additionally increases the dissolution of the pharmaceutical composition. Moreover, the increased dissolution is obtained without the requirement of providing the Erlotinib HCl in nanoparticulate form. Thus, the combination of polymorph A of Erlotinib HCl and a hydrophilic excipient in the pharmaceutical composition of the present invention results in several advantages over the prior art formulations. The first advantage is that polymorph A of Erlotinib HCl, which is known to be thermodynamically unstable, is stabilized, a further advantage is that the dissolution of the pharmaceutical composition and thereby the bioavailability of the active pharmaceutical ingredient is increased, and a further advantage is that dissolution and/or bioavailability are increased without the requirement of providing the active pharmaceutical ingredient in nanoparticulate form.

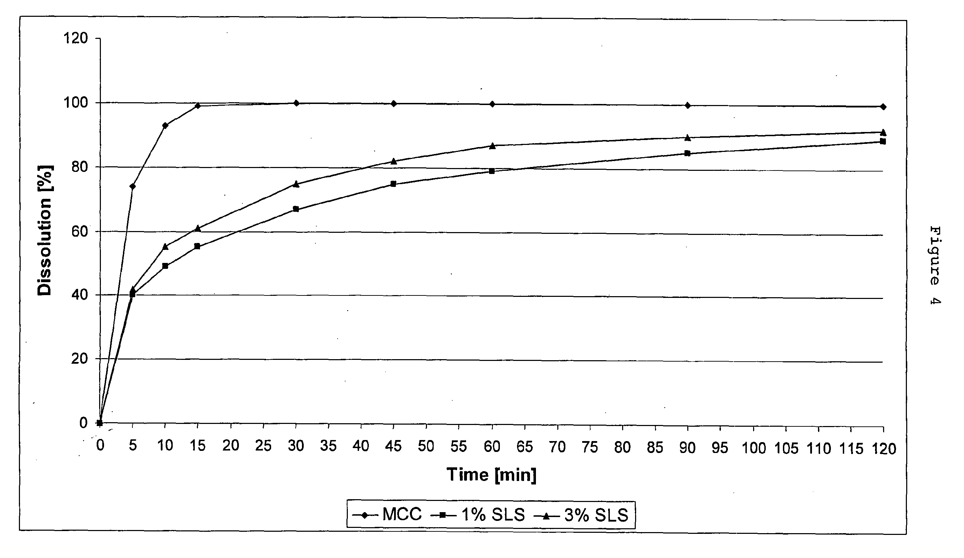

The surprising effect of the hydrophilic excipient on the dissolution profile of the pharmaceutical composition containing polymorph A of Erlotinib HCl is demonstrated by attached figure 4. This figure shows the dissolution profiles of the tablets of example 1 being according to the invention and containing microcrystalline cellulose (MCC) as hydrophilic excipient compared to tablets comprising 1 % and 3 %, respectively, of sodium lauryl sulfate (SLS), a surface stabilizer suggested in WO 2006/110811. It is evident that the hydrophilic excipient in the pharmaceutical composition of the present invention significantly increases the dissolution compared to a surface stabilizer as suggested in the prior art.

Hydrophilic excipients in the sense of the present invention contain molecules that are charge-polarized and capable of hydrogen bonding, enabling them to dissolve more readily in water than in oil or other hydrophobic solvents. A hydrophilic excipient can, for example, be a hydrophilic polymer which contains hydrophilic groups, such as hydroxy groups, amino groups, carboxy groups and/or sulfonate groups. Examples of suitable hydrophilic polymers are polysaccharides, such as microcrystalline cellulose (MCC), hydroxypropylmethyl cellulose (HPMC), carboxymethyl celluloses (CMC) (in particular sodium carboxymethyl cellulose or calcium carboxymethyl cellulose), ethyl cellulose, methyl cellulose, hydroxyethyl cellulose, ethylhydroxyethyl cellulose, and hydroxypropyl cellulose (HPC); polyvinyl pyrrolidone; polyvinyl alcohol; acrylic acid polymers and there salts; polyacryl amides; polymethacrylates; copolymers of vinylpyrrolidone and vinylacetate (such as Kollidon^{®} VA64); polyalkylene glycols, such as polypropylene glycol and polyethylene glycol; block copolymers of polyethylene glycols, such as block copolymers of polyethylene glycol and polypropylene glycol (such as Pluronic^{®}). The hydrophilic polymer can have a weight average molecular weight in the range of about 1000 g/mol to about 150000 g/mol, preferably in the range of about 2000 g/mol to about 90000 g/mol.

Microcrystalline cellulose is a preferred hydrophilic excipient in the pharmaceutical composition of the present invention. The microcrystalline cellulose can have a specific surface area in the range of about 0.78 m²/g to about 1.30 m²/g and/or a mean particle size in the range of about 10 µm to about 100 µm.

Further hydrophilic excipients useful in the pharmaceutical composition of the present invention are, for example, monosaccharides, disaccharides, oligosaccharides and sugar alcohols, such as mannitol, sorbitol, xylitol, isomalt, glucose, fructose or maltose.

The pharmaceutical composition of the present invention can contain one or more hydrophilic excipients.

In one embodiment, the hydrophilic excipient is not a surfactant, i.e. it does not contain hydrophilic and hydrophobic parts, such as the hydrophobic fatty acid residue in sodium lauryl sulfate.

In one embodiment of the present invention, the hydrophilic excipient is a hydrophilic polymer, a 2 wt.% solution of which in water exhibits a viscosity in the range of about 0.01 mPa/s to about 8 mPa/s, preferably in the range of about 0.5 mPa/s to about 7 mPa/s, more preferably in the range of about 1 mPa/s to about 6 mPa/s.

The amount of hydrophilic excipient present in the pharmaceutical composition of the present invention is not particularly limited. It can be selected by the person skilled in the art based on the specific requirements of the composition, for example with respect to its compressability. The amount must, however, be sufficient to thermodynamically stabilize polymorphic form A of Erlotinib HCl. For example, the amount can be in the range of about 10 wt.% to about 90 wt.%, preferably in the range of about 10 wt.% to about 75 wt.%, more preferably in the range of about 30 wt.% to about 75 wt.%, most preferred in the range of about 50 wt.% to about 70 wt.%, each based on the total weight of the pharmaceutical composition.

In order to provide a pharmaceutical composition having an optimum dissolution profile, it is preferred that the pharmaceutical composition is essentially free of polymorph B of Erlotinib HCl. Being essentially free of polymorph B means that polymorph B is present in the pharmaceutical composition of the present invention in an amount of less than 5 wt.%, preferably in an amount of less than 1 wt.%, each based on the total amount of Erlotinib HCl. Most preferable, the pharmaceutical composition of the present invention does not contain any polymorph B of Erlotinib HCl. Furthermore, it is preferred that the pharmaceutical composition does not contain any other polymorph of Erlotinib HCl than polymorph A. Thus, the pharmaceutical composition of the present invention preferably comprises Erlotinib HCl essentially only as polymorph A, i.e. at least 95 wt.% of the total amount of Erlotinib HCl in the pharmaceutical composition is present in polymorphic form A, preferably at least 99 wt.%. Most preferred Erlotinib HCl is present in the pharmaceutical composition only in polymorphic form A.

The particle size of the active pharmaceutical ingredient in the pharmaceutical composition of the present invention is not particularly limited. However, in view of the disadvantages associated with the preparation and handling of nanoparticles, it is preferred that the Erlotinib HCl in the pharmaceutical composition of the present invention has a D50 particle size of above 2 µm. D50 particle size means a particle size distribution where 50 vol.% of the total amount of Erlotinib HCl in the pharmaceutical composition has a particle size of above 2 µm when measured with a Master Sizer 2000 using propan-2-ol as dispersant.

It was further found that the advantageous effects of the hydrophilic excipient on the thermodynamic stability of polymorph A of Erlotinib HCl and the dissolution of the pharmaceutical composition are particularly strong when the surface stabilizer is absorbed to or associated with the surface of the Erlotinib HCl particles. This absorption or association can be obtained by intimate mixing polymorph A of Erlotinib HCl and the hydrophilic excipient, for example, by milling, grinding, or triturating.

Therefore, the present invention also relates to a process for the preparation of a pharmaceutical composition as defined above, comprising the step of intimate mixing polymorph A of Erlotinib HCl and a hydrophilic excipient. Preferably the step of intimate mixing is carried out by milling, grinding, or triturating. The process can comprise the further steps of adding additional excipients and forming a solid dosage form, such as by compression, in particular by dry compaction.

The pharmaceutical composition of the present invention can additionally comprise one or more further active pharmaceutical ingredients, in particular one or more compounds useful in treating hyperproliferative disorders, such as cancer or other neoplastic diseases, or the pharmaceutical composition of the present invention can be administered in conjunction with such a compound. Examples of suitable other active pharmaceutical ingredients are disclosed, for example, in WO 2006/110811.

The dosage of Erlotinib HCl can vary within wide limits depending on the disease to be controlled, the age and the individual condition of the patient and the mode of administration, and will, of course, be fit to the individual requirements in each particular case. For adult patients a daily dosage of about 1 mg to about 1000 mg, especially about 5 mg to about 500 mg, preferably about 25 mg to about 200 mg is applicable. Depending on severity of the disease and the precise pharmacokinetic profile, the compound can be administered with one or several daily dosage units, e.g. in one to three dosage units.

The pharmaceutical composition of the present invention may further comprise one or more pharmaceutically acceptable excipients, such as fillers, binding agents, lubricants, flow enhancers, antisticking agents, disintegrating agents and solubilizers. As pharmaceutically acceptable excipients conventional excipients known to the person skilled in the art may be used. See for example "Lexikon der Hilfsstoffe für Pharmazie, Kosmetik und angrenzende Gebiete", edited by H. P. Fiedler, 4th Edition, Edito Cantor; Aulendorf and earlier editions, and "Handbook of Pharmaceutical Excipients", Third Edition, edited by Arthur H. Kibbe, American Pharmaceutical Association, Washington, USA, and Pharmaceutical Press, London.

Preferred examples of the fillers are lactose, mannitol, sorbitol or microcrystalline cellulose. The filler is suitably present in an amount of about 0 wt.% to about 30 wt.% of the total weight of the composition.

The binding agent can for example be microcrystalline cellulose (MCC) or hydroxypropylmethyl cellulose (HPMC). Preferably, the binding agent is present in an amount of about 1 wt.% to about 25 wt.%, more preferably at about 2 wt.% to about 10 wt.% of the total weight of the composition.

The lubricant is preferably a stearate such as earth alkali metal stearate, such as magnesium stearate or sodium stearyl fumarate. The lubricant is suitably present in an amount of about 0.5 wt.% to about 2.5 wt.% of the total weight of the composition.

Suitable disintegrating agents are for example cellulose derivatives such croscarmellose sodium, starch derivatives such as sodium carboxymethyl starch, pregelatinized starch, synthetic polymers such as cross-linked polyvinylpyrrolidone (crospovidone) or sodium bicarbonate. The disintegrant or disintegrant mixture is suitably present in an amount of about 1 wt.% to about 20 wt.%, more preferably at about 5 wt.% to about 10 wt.% of the total weight of the composition.

The flow enhancer can for example be colloidal silicon dioxide. Preferably, the flow enhancer is present in an amount of about 0.5 wt.% to about 8 wt.%, more preferably at about 1 wt.% to about 4 wt.% of the total weight of the composition.

The antisticking agent is for example talcum and may be present in amounts of about 1 wt.% to about 5 wt.%, more preferably in an amount of about 1.5 wt.% to about 3 wt.% of the total weight of the composition.

An improvement of the solubility of the active pharmaceutical ingredient can for example be achieved by the addition of complex forming agents/compounds (e.g. sodium benzoate, sodium salicylate or cyclodextrins), the alternation of solvent properties (e.g. by adding PVP or polyethylene glycols) or the addition of solubilizers which form tenside micelles (e.g. surfactants).

Suitable solubilizers are for example surfactants such as polyoxyethylene alcohol ethers (e.g. Brij®), polysorbates (e.g. Tween®) or polyoxypropylene polyoxyethylene copolymers (poloxamer; e.g. Pluronic®) and may be present in amounts of about 0.5 wt.% to about 7 wt.%, more preferably about 1 wt.% to about 5 wt.% of the total weight of the composition.

Alternatively, a pseudo-emulsifier can be used. Its mechanism of action mainly relies on an enhancement of viscosity. However, pseudo-emulsifiers also possess emulsifying properties.

Preferred pseudo-emulsifiers are for example cellulose ethers, gum Arabic or tragacanth. Pseudo-emulsifiers may be present in amounts of about 1 wt.% to about 10 wt.%, more preferably about 3 wt.% to about 7 wt.% of the total weight of the composition.

A person skilled in the art may use these or other excipients depending on the selected process of preparing the pharmaceutical composition of the invention.

The pharmaceutical composition of the present invention can be formulated in any known manner, preferably as tablets, capsules, granules, pellets or sachets. A particularly preferred pharmaceutical composition is in the form of tablets. The pharmaceutical composition may contain for example dosage amounts of 25, 100 or 150 mg of Erlotinib. Thus the administered amount can be readily varied according to individual tolerance and safety.

The pharmaceutical composition of the present invention can be manufactured according to standard methods known in the art. Granulates according to the invention can be obtained by dry compaction or wet granulation. These granulates can subsequently be mixed with e.g. suitable disintegrating agents, glidants and lubricants and the mixture can be compressed into tablets or filled into sachets or capsules of suitable size.

Tablets can also be obtained by direct compression of a suitable powder mixture, i.e. without any preceding granulation of the excipients.

Suitable powder or granulate mixtures according to the invention are also obtainable by spray drying, lyophilization, melt extrusion, pellet layering, coating of the active pharmaceutical ingredient or any other suitable method. The so obtained powders or granulates can be mixed with one or more suitable ingredients and the resulting mixtures can either be compressed to form tablets or filled into sachets or capsules.
The above mentioned methods known in the art also include grinding and sieving techniques permitting the adjustment of desired particle size distributions.

### Figures

Figure 1 shows the XRPD pattern of polymorph A of Erlotinib HCl taken from WO 01/34574 A1.
Figure 2 shows the XRPD patterns of a mixture of 90 % polymorph A and 10 % polymorph B of Erlotinib HCl at time 0, after 4 weeks and after 12 weeks (from bottom to top).
Figure 3 shows the XRPD patterns of grounded tablets according to the present invention at time 0, after 4 weeks and after 12 weeks (from bottom to top).
Figure 4 shows the dissolution profile of a tablet according to the present invention comprising MCC as hydrophilic excipient, compared to the dissolution profiles of tablets comprising 1 wt.% and 3 wt.% sodium lauryl sulfate (SLS), respectively.

The following example further illustrates the invention but is not to be construed as limiting.

### Example

Tablets where prepared with the ingredients according to table 2

**Table 2**

| **Ingredient** | **amount [mg/tablet]** |
|---|---|
| Erlotinib HCl Polymorph A | 27.50 |
| MCC (Avicel PH 101) | 29.25 |
| Sodium lauryl sulfate | 1.00 |
| PVP (Kollidon CL) | 12.00 |
| MCC (Avicel PH 102) | 29.25 |
| Magnesium stearate | 1.25 |

Avicel PH 101, Erlotinib HCl Polymorph A, sodium lauryl sulfate and Kollidon CL where mixed and triturated before sieving through a 125 µm sieve. The mixture was pressed, crushed and sieved over a 630 µm sieve. The remaining excipients were added, the mixture was again mixed and pressed into tablets.

The dissolution of the obtained tablets was measured using the paddle method (USP app. II) in 1000 ml 0.1 N HCl, pH 1 at 37°C and 100 rpm with the addition of 0.5 % sodium lauryl sulfate. The dissolution profile was measured after storage for two and four weeks, respectively, whereby the storage conditions were 40°C/75 % relative humidity and 25°C/60 % relative humidity, respectively. The results of the dissolution tests are summarized in table 3.

**Table 3**

| **Dissolution** | **2 weeks** | | **4 weeks** | |
|---|---|---|---|---|
| | 40°C/75 % RH | 25°C/60 % RH | 40°C/75 % RH | 25°C/60 % RH |
| after 5 min. [%] | 54.3 | 55.0 | 55.4 | 58.7 |
| after 15 min. [%] | 89.0 | 91.6 | 88.7 | 97.5 |
| after 60 min. [%] | 100.0 | 100.0 | 100.0 | 100.0 |

The dissolution profile of the tablets after storage for four weeks at 40°C and 75 % relative humidity is also shown in figure 4. The dissolution profile of the tablets of the present invention is excellent and remains stable during storage even under harsh conditions.

## Claims

1. Pharmaceutical composition comprising polymorph A of N-(3-ethynylphenyl)-6,7-bis(2-methoxyethoxy)-4-quinazolinamine hydrochloride in a thermodynamically stable form.

2. Pharmaceutical composition according to claim 1, further comprising a hydrophilic excipient.

3. Pharmaceutical composition according to claim 2, wherein the hydrophilic excipient is selected from the group consisting of hydrophilic polymers, monosaccharides, disaccharides, oligosaccharides and sugar alcohols.

4. Pharmaceutical composition according to claim 3, wherein the hydrophilic excipient is selected from the group consisting of polysaccharides, such as microcrystalline cellulose, hydroxypropylmethyl cellulose, carboxymethyl celluloses, ethyl cellulose, methyl cellulose, hydroxyethyl cellulose, ethylhydroxyethyl cellulose, and hydroxypropyl cellulose; polyvinyl pyrrolidone; polyvinyl alcohol; acrylic acid polymers and there salts; polyacryl amides; polymethacrylates; copolymers of vinylpyrrolidone and vinylacetate; polyalkylene glycols, such as polypropylene glycol and polyethylene glycol; block copolymers of polyethylene glycols, such as block copolymers of polyethylene glycol and polypropylene glycol; mannitol; sorbitol; xylitol; isomalt; glucose; fructose; maltose; and mixtures thereof.

5. Pharmaceutical composition according to claim 4, wherein the hydrophilic excipient is microcrystalline cellulose having a specific surface area in the range of about 0.78 m²/g to about 1.30 m²/g and/or a mean particle size in the range of about 10 µm to about 100 µm.

6. Pharmaceutical composition according to any of the preceding claims, being essentially free of polymorph B of N-(3-ethynylphenyl)-6,7-bis(2-methoxyethoxy)-4-quinazolinamine hydrochloride.

7. Pharmaceutical composition according to any of the preceding claims, wherein the N-(3-ethynylphenyl)-6,7-bis(2-methoxyethoxy)-4-quinazolinamine hydrochloride has a D50 particle size of above 2 µm.

8. Pharmaceutical composition according to any of the preceding claims, wherein the surface stabilizer is absorbed to or associated with the surface of N-(3-ethynylphenyl)-6,7-bis(2-methoxyethoxy)-4-quinazolinamine hydrochloride particles.

9. Pharmaceutical composition according to any of the preceding claims, being in the form of a solid dosage form, such as tablets, capsules, granules, pellets and sachets.

10. Pharmaceutical composition according to any of the preceding claims, obtainable by a process comprising the step of intimate mixing polymorph A of N-(3-ethynylphenyl)-6,7-bis(2-methoxyethoxy)-4-quinazolinamine hydrochloride and a hydrophilic excipient.

11. Pharmaceutical composition according to claim 10, wherein the step of intimate mixing is carried out by milling, grinding, or triturating.

12. Process for the preparation of a pharmaceutical composition as defined in any of claims 1-11, comprising the step of intimate mixing polymorph A of N-(3-ethynylphenyl)-6,7-bis(2-methoxyethoxy)-4-quinazolinamine hydrochloride and a hydrophilic excipient.

13. Process according to claim 12, wherein the step of intimate mixing is carried out by milling, grinding, or triturating.

14. Process according to claim 12 or 13, comprising the further steps of adding additional excipients and forming a solid dosage form, such as by compression, in particular by dry compaction.

15. Use of a hydrophilic excipient for stabilizing the polymorphic form of polymorph A of N-(3-ethynylphenyl)-6,7-bis(2-methoxyethoxy)-4-quinazolinamine hydrochloride.
